(19) Europäisches Patentamt
European Patent Office
Office européen des brevets

(11) EP 3 632 407 A1

(12) # EUROPEAN PATENT APPLICATION
published in accordance with Art. 153(4) EPC

(43) Date of publication:
**08.04.2020 Bulletin 2020/15**

(21) Application number: **18809289.4**

(22) Date of filing: **30.05.2018**

(51) Int Cl.:
***A61K 8/68*** *(2006.01)* ***A61K 8/06*** *(2006.01)*
***A61K 8/31*** *(2006.01)* ***A61K 8/37*** *(2006.01)*
***A61K 8/73*** *(2006.01)* ***A61K 8/894*** *(2006.01)*
***A61Q 19/00*** *(2006.01)*

(86) International application number:
**PCT/JP2018/020797**

(87) International publication number:
**WO 2018/221603 (06.12.2018 Gazette 2018/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.05.2017 JP 2017107246**
**04.12.2017 JP 2017232353**

(71) Applicant: **Kao Corporation**
**Chuo-Ku**
**Tokyo 103-8210 (JP)**

(72) Inventor: **TAIMA, Hidetoshi**
**Odawara-shi**
**Kanagawa 250-0002 (JP)**

(74) Representative: **Hoffmann Eitle**
**Patent- und Rechtsanwälte PartmbB**
**Arabellastraße 30**
**81925 München (DE)**

(54) **WATER-IN-OIL EMULSION COMPOSITION**

(57) The present invention provides a water-in-oil emulsion composition having a high water occlusive of a coating film and having a good skin protective feeling and glossiness.

A water-in-oil emulsion composition is provided containing the following components (A), (B), (C), (D), (E) and (F) : (A) 1% by mass or more and 30% by mass or less of an oily component having a melting point of from 50 to 150°C; (B) 0.5% by mass or more and 8% by mass or less of a polyglycerol fatty acid ester; (C) a lipophilic surfactant other than component (B), having an HLB of from 2 to 6; (D) 0.15% by mass or more and 8% by mass or less of a dextrin fatty acid ester having 8 to 22 carbon atoms in the fatty acid moiety; (E) a hydrocarbon oil that is liquid at 25°C; and (F) water.

EP 3 632 407 A1

## Description

[Field of the Invention]

The present invention relates to a water-in-oil emulsion composition.

[Background of the invention]

Emulsion compositions used as skin cosmetics and quasi-drugs are roughly classified into oil-in-water emulsion compositions and water-in-oil emulsion compositions. Among these, the water-in-oil emulsion compositions have excellent moisture retention because the outer phase is an oil phase but is sticky, causing a problem in terms of the feeling upon use. There is a report of a technique of blending a specific gelling agent with a polyglycerol fatty acid ester and/or polyoxyethylene hydrogenated castor oil as an emulsifying agent in order to improve the feeling upon use of the water-in-oil emulsion composition (Patent Document 1). In addition, there are reports of a technique of blending a phytosterol derivative and a dextrin fatty acid ester (Patent Document 2), and a technique of blending ceramide, an organically modified clay mineral, a diester of N-acylglutamic acid and a phytosterol (Patent Document 3) in order to obtain a highly stable external composition containing a high concentration of ceramides, which are oily components that is solid at normal temperature and having a skin barrier function.

(Patent Document 1) WO 99/25310 A1
(Patent Literature 2) JP-A-2016-190816
(Patent Document 3) JP-A-2010-513221

[Summary of the Invention]

The present invention provides a water-in-oil emulsion composition, comprising the following components (A), (B), (C), (D), (E) and (F):

(A) 1% by mass or more and 30% by mass or less of an oily component having a melting point of from 50 to 150°C;
(B) 0.5% by mass or more and 8% by mass or less of a polyglycerol fatty acid ester;
(C) a lipophilic surfactant other than component (B), having an HLB of from 2 to 6;
(D) 0.15% by mass or more and 8% by mass or less of a dextrin fatty acid ester having 8 to 22 carbon atoms in the fatty acid moiety;
(E) a hydrocarbon oil that is liquid at 25°C; and
(F) water.

[Detailed Description of the Invention]

In the techniques of Patent Documents 1 to 3, the water occlusive of a film obtained by applying the preparation is not sufficiently exhibited, and they are not satisfactory in terms of providing protective feeling and glossiness when applying the preparation to the skin and a good feeling upon use of the skin applied without stickiness and uncomfortable feeling.
Accordingly, the present invention provides a water-in-oil emulsion composition which contains an oily component that is solid at normal temperature, is excellent in water occlusive, skin protective feeling, and glossiness, and has a good feeling upon use of the skin applied without stickiness and uncomfortable feeling.

Therefore, the present inventor blended various components in a water-in-oil composition containing an oily component that is solid at room temperature, and studied water occlusive, protective feeling and glossiness and the like. As a result, the present inventor found that when a polyglycerol fatty acid ester, a lipophilic surfactant having an HLB of from 2 to 6, a dextrin fatty acid ester, and a hydrocarbon oil that is liquid at 25°C are blended in a specified ratio, the water-in-oil emulsion composition which is excellent in not only water occlusive but also skin protective feeling and glossiness, provides a good feeling upon use of the applied skin without stickiness and uncomfortable feeling, and has a good storage stability is obtained, thereby completing the present invention.

The water-in-oil emulsion composition of the present invention has excellent water occlusive or a barrier function, is excellent in skin protective feeling and glossiness, has good feeling upon use without stickiness or uncomfortable feeling of the applied skin, and is excellent in stability without separation of the emulsion even when stored for a long period of time. In the water-in-oil emulsion composition of the present invention, when component (A) is a ceramide in particular, it is confirmed by microscopic observation that crystals are present in the preparation in a refined or amorphous

state, and it is considered that high water occlusive of a film obtained by applying the preparation is caused by being such a refined or amorphous state.

In the water-in-oil emulsion composition of the present invention, an oily component having a melting point of from 50 to 150°C is used as component (A). The oily component having a melting point of from 50 to 150°C is an oily component that is solid at normal temperature (from 5 to 35°C). Examples of the solid oily component include a sphingolipid such as a ceramide and a sphingosine (including a natural or synthetic one); a sterol and an analogue thereof such as cholesterol, dehydrocholesterol, β-sitosterol, stearoyl cholesteryl ester, isostearoyl cholesteryl ester, and vegetable oil fatty acid cholesteryl ester; a $C_{16}$-$C_{22}$ fatty acid such as stearic acid and behenic acid; and a $C_{16}$-$C_{22}$ alcohol and an analogue thereof such as cetyl alcohol, stearyl alcohol, behenyl alcohol, batyl alcohol, and kimyl alcohol.

Examples of a ceramide include a structural analogue of ceramide described in JP-A-S62-228048, JP-A-S63-216812, JP-A-S63-227513, JP-A-S64-29347, JP-A-S64-31752, and JP-A-H8-319263, in addition to a natural ceramide and a sphingosine derivative. Specifically, a compound selected from compounds of the following formulae (1) and (2) is preferable from the viewpoint of imparting skin-covering feeling and glossiness to the skin. A compound of formula (1) is particularly preferable.

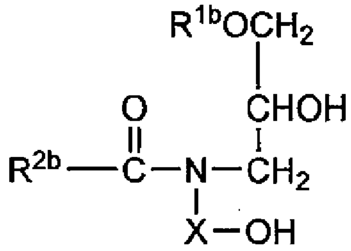

(1)

wherein $R^{1b}$ represents a hydrocarbon group having 10 to 26 carbon atoms, $R^{2b}$ represents a hydrocarbon group having 9 to 25 carbon atoms, and X represents $-(CH_2)_n-$ (where n represents an integer of from 2 to 6).

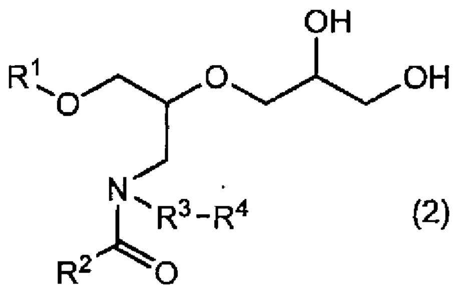

(2)

wherein $R^1$ and $R^2$ are the same or different from each other and represent an optionally hydroxylated hydrocarbon group having 1 to 40 carbon atoms, $R^3$ represents an alkylene group having 1 to 6 carbon atoms or a single bond, $R^4$ represents a hydrogen atom, an alkoxy group having 1 to 12 carbon atoms, or 2,3-dihydroxypropyloxy group (where $R^4$ is a hydrogen atom when $R^3$ is a single bond.)

In the above formulae (1) and (2), an alkyl group or an alkenyl group is preferable as the hydrocarbon group.

Examples of the compound of formula (1) include N-(hexadecyloxyhydroxypropyl)-N-hydroxyethylhexadecanamide, and examples of the compound of formula (2) include long chain dibasic acid bis 3-methoxypropylamide. Among these components (A), a ceramide (in particular, a natural ceramide, a sphingosine, a compound of the above formulae (1) and (2)), a $C_{16}$-$C_{22}$ fatty acid, and a $C_{16}$-$C_{22}$ alcohol are preferable; a ceramide is more preferable; a compound selected from the above formulae (1) and (2) is even more preferable; and a compound of the above formula (1) is even more preferable from the viewpoint of water occlusive and skin protective feeling. These solid oily components can be used singly or in combination of two or more of them.

Component (A) is contained in the water-in-oil emulsion composition of the present invention in an amount of 1% by mass or more and 30% by mass or less. If the content of component (A) is within this range, sufficient water occlusive is obtained, skin protective feeling is good, and feeling upon use of the applied skin is good without stickiness and uncomfortable. The content of component (A) is preferably 1.5% by mass or more, more preferably 2% by mass or more, even more preferably 3% by mass or more, and even more preferably 5% by mass or more; preferably 20% by mass or less, more preferably 18% by mass or less, and even more preferably 16% by mass or less from the same viewpoint. The specific range is preferably 1.5% by mass or more and 20% by mass or less, more preferably 2% by mass or more and 18% by mass or less, even more preferably 3% by mass or more and 18% by mass or less, and even

more preferably 5% by mass or more and 16% by mass or less.

Component (B) is a polyglycerol fatty acid ester. The polyglycerol fatty acid ester preferably has the polyglycerol moiety of a condensate of 2 to 20 glycerins, and the fatty acid moiety is preferably a $C_8$-$C_{24}$ fatty acid from the viewpoint of water occlusive, protective feeling of the skin, glossiness, storage stability, and good feeling upon use of the applied skin without stickiness or uncomfortable feeling. Among these polyglycerol fatty acid esters, a polyglycerol difatty acid ester and a polyglycerol trifatty acid ester are preferable and a polyglycerol difatty acid ester is more preferable from the viewpoints of achieving water occlusive, skin protective feeling, glossiness, storage stability, and good feeling upon use without stickiness or uncomfortable feeling when applying to the skin. The fatty acid moiety is preferably a $C_{14}$-$C_{22}$ fatty acid, more preferably a $C_{16}$-$C_{22}$ fatty acid, and even more preferably oleic acid, isostearic acid, stearic acid, hydroxy stearic acid, and behenic acid, even more preferably isostearic acid and stearic acid, and even more preferably isostearic acid.

More specifically, a polyglycerol di $C_{16}$-$C_{22}$ fatty acid ester and a polyglycerol tri $C_{16}$-$C_{22}$ fatty acid ester are preferable, polyglycerol diisostearate and polyglycerol triisostearate are more preferable, and polyglycerol diisostearate ester is more preferable.

The polyglycerol moiety of these polyglycerol fatty acid esters is preferably a condensate of 2 to 12 glycerins, more preferably a condensate of 2 to 10 glycerins, even more preferably a condensate of 2 to 3 glycerins, and even more preferably a condensate of 2 glycerins, from the viewpoint of water occlusive, skin protective feeling, glossiness, storage stability, and good feeling upon use of the applied skin without stickiness or uncomfortable feeling.

Component (B) is contained in the water-in-oil emulsion composition of the present invention in an amount of 0.5% by mass or more and 8% by mass or less. If the content of component (B) is within this range, sufficient water occlusive can be obtained, protective feeling and glossiness of the skin are also good, good feeling upon use of the skin can be obtained without stickiness and uncomfortable feeling, and long-term storage stability is excellent. The content of component (B) is preferably 0.7% by mass or more, more preferably 0.8% by mass or more, and even more preferably 1% by mass or more; preferably 7% by mass or less, more preferably 6% by mass or less, even more preferably 5% by mass or less, and even more preferably 3% by mass or less from the same viewpoint. The specific range is preferably 0.7% by mass or more and 7% by mass or less, more preferably 0.8% by mass or more and 6% by mass or less, even more preferably 1% by mass or more and 5% by mass or less, and even more preferably 1% by mass or more and 3% by mass or less.

Component (C) is a lipophilic surfactant other than component (B), having an HLB of 2 to 6. In the water-in-oil emulsion composition of the present invention, when two kinds of surfactants of component (B) and component (C) are used in combination, while maintaining excellent water occlusive, skin protective feeling, skin-covering feeling, and glossiness is enhanced, non-sticky and non-uncomfortable feeling is excellent, and the long-term storage stability is also excellent.

As the lipophilic surfactant having an HLB of from 2 to 6, a nonionic surfactant having an HLB in the range of from 2 to 6 is preferable, and examples thereof include a glycerol fatty acid ester, a propylene glycol fatty acid ester, a sorbitan fatty acid ester, a polyoxyethylene castor oil, a polyoxyethylene hydrogenated castor oil, a polyoxyethylene hydrogenated castor oil fatty acid ester, a polyethylene glycol fatty acid ester, an alkyl glyceryl ether, an alkyl polyglyceryl ether, a polyoxyethylene alkyl ether, a polyoxyethylene alkyl ether fatty acid ester, a polyoxyethylene alkyl amine, a silicone surfactant, and the like. Examples of the silicone surfactant include a polyether modified silicone, a polyether/alkyl co-modified silicone, a polyglycerol modified silicone, a polyglycerol/alkyl co-modified silicone, an alkyl/polyether modified silicone, an alkyl silicone dendron polyether modified silicone, and the like. The silicone chain is preferably a linear type, a branched chain type, and a type where silicone chains are crosslinked. These are commercially available from Shin-Etsu Chemical Co., Ltd, Dow Corning Toray Co., Ltd, Momentive Performance Materials Japan Inc., Wacker Asahikasei Silicone Co., Ltd and the like.

From the viewpoint of particularly improving emulsifying property, long-term storage stability and water occlusive, and adjusting the viscosity to a suitable level and from the viewpoint of achieving good feeling upon use without stickiness or uncomfortable feeling when applied to the skin, component (C) is preferably a silicone surfactant, a polyoxyethylene hydrogenated castor oil, an alkyl glyceryl ether and a sorbitan fatty acid ester; more preferably a silicone surfactant, a polyoxyethylene hydrogenated castor oil and an alkyl glyceryl ether, and even more preferably a silicone surfactant and an alkyl glyceryl ether.

The silicone surfactant is preferably a polyether modified silicone, a polyglycerol modified silicone, a polyether/alkyl co-modified silicone, and a polyglycerol/alkyl co-modified silicone, and more preferably a polyether modified silicone. The silicone chain is preferably a linear type, a branched chain type, and a crosslink type, more preferably a linear type and a branched chain type, even more preferably a linear type. More specifically, a polyether modified silicone having a linear silicone chain, a polyether modified silicone having a branched silicone chain, a polyether/alkyl co-modified silicone having a linear silicone chain, and a polyether/alkyl co-modified silicone having a branched silicone chain are preferable; a polyether modified silicone having a linear silicone chain, and a polyether modified silicone having a branched silicone chain are more preferable; a polyether modified silicone having a linear silicone chain is even more

preferable.

As component (C), polyoxyethylene hydrogenated castor oil, alkyl glyceryl ether, polyether modified silicone having a linear silicone chain, and a polyether modified silicone having a branched silicone chain are preferable; and alkyl glyceryl ether, and a polyether modified silicone having a linear silicone chain are preferable from the viewpoint of particularly improving emulsifying property, long-term storage stability and water occlusive, and adjusting the viscosity to a suitable level, and from the viewpoint of providing a good feeling upon use without a sticky feeling and uncomfortable feeling when applied to the skin, and polyether modified silicone having a branched chain is more preferable.

One or more of these can be used, and it is preferable to use two or more of them from the viewpoint of improving emulsifying property and long-term storage stability, maintaining excellent water occlusive, being excellent in skin protective feeling, skin-covering feeling, and glossiness, and achieving good feeling upon use without sticky feeling or uncomfortable feeling.

Commercially available products thereof include a polyether modified silicone such as silicone BY11-030 (PEG/PPG-19/19 Dimethicone) (Dow Corning Toray Co., Ltd), silicone BY22-008M (PEG/PPG-19/19 Dimethicone) (Dow Corning Toray Co., Ltd), silicone SH3775M (PEG-12 Dimethicone) (Dow Corning Toray Co., Ltd), silicone KF-6015 (PEG-3 dimethicone) (Shin-Etsu Chemical Co., Ltd), silicone KF-6017 (PEG-10 dimethicone) (Shin-Etsu Chemical Co., Ltd), silicone KF-6028 (PEG-9 polydimethylsiloxyethyl dimethicone) (Shin-Etsu Chemical Co., Ltd), etc.; a polyoxyethylene hydrogenated castor oil such as EMALEX HC-5 (PEG-5 hydrogenated castor oil) (Nihon Emulsion Co., Ltd.), and EMALEX RWIS-315 (PEG-15 hydrogenated castor oil triisostearate) (Nihon Emulsion Co., Ltd.); and isostearyl glyceryl ether. Among these, silicone BY11-030 (PEG/PPG - 19/19 Dimethicone), silicone SH3775M (PEG-12 Dimethicone), silicone KF-6015 (PEG-3 dimethicone), silicone KF-6017 (PEG-10 dimethicone), silicone KF-6028 (PEG-9 polydimethylsiloxyethyl dimethicone), EMALEX HC-5 (PEG-5 hydrogenated castor oil), and isostearyl glyceryl ether are more preferable; and silicone KF-6015 (PEG-3 dimethicone), silicone KF-6017 (PEG-10 dimethicone), and isostearyl glyceryl ether are even more preferable. One or more of them may be used.

HLB (Hydrophilic-Lipophilic Balance) herein indicates the molecular weight of the hydrophilic group moiety in the total molecular weight of the surfactant, and HLB is obtained by the equation of Griffin.

From the viewpoint of improving emulsifying property, long-term storage stability, and water occlusive, adjusting the viscosity to a suitable level, and obtaining good feeling upon use of the applied skin without sticky feeling and uncomfortable feeling, the content of component (C) is preferably 0.5% by mass or more, more preferably 0.8% by mass or more, and even more preferably 1% by mass or more; and preferably 8% by mass or less, more preferably 7% by mass or less, even more preferably 5% by mass or less, even more preferably 3.5% by mass or less, and even more preferably 2% by mass or less in the water-in-oil emulsion composition of the present invention. The specific range is preferably 0.5% by mass or more and 8% by mass or less, more preferably 0.8% by mass or more and 7% by mass or less, even more preferably 1% by mass or more and 5% by mass or less, even more preferably 1% by mass or more and 3.5% by mass or less, and even more preferably 1% by mass or more and 2% by mass or less.

From the viewpoint of improving emulsifying property, long-term storage stability, and water occlusive, adjusting the viscosity to a suitable level, and obtaining good feeling upon use of the applied skin without sticky feeling and uncomfortable feeling, the total content of components (B) and (C) is preferably 0.5% by mass or more, more preferably 1.0% by mass or more, even more preferably 1.5% by mass or more, even more preferably 2% by mass or more, and even more preferably 2.5% by mass or more; and preferably 15% by mass or less, more preferably 12% by mass or less, even more preferably 10% by mass or less, even more preferably 8% by mass or less, even more preferably 6% by mass or less, even more preferably 5% by mass or less, and even more preferably 4.5% by mass or less in the water-in-oil emulsion composition of the present invention. The specific range is preferably 0.5% by mass or more and 12% by mass or less, more preferably 1.5% by mass or more and 10% by mass or less, even more preferably 2% by mass or more and 8% by mass or less, even more preferably 2% by mass or more and 6% by mass or less, even more preferably 2.5% by mass or more and 5% by mass or less, and even more preferably 2.5% by mass or more and 4.5% by mass or less.

Component (D) is a dextrin fatty acid ester having 8 to 22 carbon atoms in the fatty acid moiety. By containing such a dextrin fatty acid ester, the water occlusive of a film obtained by applying the preparation can be improved, protective feeling of the skin, skin covering feeling, and storage stability can be also improved, and the viscosity can be adjusted to a desired level. In particular, in the case where component (A) is a ceramide, it is found that the crystal size of component (A) in the water-in-oil emulsion composition becomes particularly small or amorphous by coexistence of component (D), and the water occlusive of a film obtained by applying the preparation and protective feeling of the skin are remarkably improved. As the dextrin fatty acid ester of component (D), a dextrin fatty acid ester having 12 to 18 carbon atoms in the fatty acid moiety is more preferable, a dextrin fatty acid ester having 14 to 18 carbon atoms in the fatty acid moiety is more preferable, a dextrin fatty acid ester having 14 to 16 carbon atoms in the fatty acid moiety is even more preferable, and a dextrin fatty acid ester having 16 carbon atoms in the fatty acid moiety is even more preferable. Specific examples thereof include dextrin caprylate, dextrin caprate, dextrin laurate, dextrin ethylhexanoate, dextrin myristate, dextrin palmitate, dextrin stearate, dextrin isostearate, dextrin behenate, and the like, and preferably

dextrin ethylhexanoate, dextrin myristate, dextrin palmitate, and dextrin stearate, more preferably dextrin myristate, and dextrin palmitate, and even more preferably dextrin palmitate; and one or more of these may be used. It should be noted that two or more of dextrin fatty acid esters prepared in advance may be used, or a mixed fatty acid dextrin ester obtained by mixing fatty acids at the time of production and then reacting with dextrin may be used.

Component (D) is contained in the water-in-oil emulsion composition of the present invention in an amount of 0.15% by mass or more and 8% by mass or less. If the content of component (D) falls within this range, sufficient water occlusive can be obtained, the skin protective feeling and glossiness are good, and long-term storage stability is excellent. The content of component (D) is preferably 0.2% by mass or more, more preferably 0.3% by mass or more, and even more preferably 0.4% by mass or more; and preferably 6% by mass or less, more preferably 5% by mass or less, even more preferably 4% by mass or less, and even more preferably 2% by mass or less from the same viewpoint. The specific range is preferably 0.2% by mass or more and 6% by mass or less, more preferably 0.3% by mass or more and 5% by mass or less, even more preferably 0.4% by mass or more and 4% by mass or less, and even more preferably 0.4% by mass or more and 2% by mass or less.

Component (E) is a hydrocarbon oil that is liquid at 25°C. By blending component (E) and components (B) to (D) in addition to component (A) (solid oily component), not only the water occlusive is improved, but also the moisture retention and the glossiness of the skin are improved, and long-term storage stability is also improved.

Such hydrocarbon oils include a linear or branched hydrocarbon oil such as liquid paraffin, liquid isoparaffin, squalane and squalene, and are preferably squalane. One or more of them may be used.

From the viewpoint of increased water occlusive, skin protective feeling, skin-covering feeling, and glossiness, good feeling upon use without sticky feeling and uncomfortable feeling and long-term storage stability, the content of component (E) is preferably 1% by mass or more, more preferably 3% by mass or more, even more preferably 4% by mass or more, even more preferably 5% by mass or more, and even more preferably 7% by mass or more; and preferably 30% by mass or less, more preferably 20% by mass or less, even more preferably 18% by mass or less, even more preferably 16% by mass or less, even more preferably 15% by mass or less, even more preferably 12% by mass or less, and even more preferably 10% by mass or less in the water-in-oil emulsion composition of the present invention. The specific range is preferably 1% by mass or more and 30% by mass or less, more preferably 3% by mass or more and 30% by mass or less, even more preferably 3% by mass or more and 20% by mass or less, even more preferably 4% by mass or more and 18% by mass or less, even more preferably 5% or more and 16% by mass or less, even more preferably 7% or more and 15% by mass or less, even more particularly preferably 7% or more and 12% by mass or less, and even more preferably 7% or more and 10% by mass or less.

The water-in-oil emulsion composition of the present invention contains (F) water as an aqueous phase component. From the viewpoint of water occlusive, skin protective feeling, glossiness and long-term storage stability, the content of water is preferably 10% by mass or more, more preferably 15% by mass or more, and even more preferably 20% by mass or more; and preferably 65% by mass or less, more preferably 60% by mass or less, and even more preferably 50% by mass or less in the water-in-oil emulsion composition of the present invention. The specific range is preferably 10% by mass or more and 65% by mass or less, more preferably 15% by mass or more and 60% by mass or less, and even more preferably 20% by mass or more and 50% by mass or less.

The water-in-oil emulsion composition of the present invention contains (G) one or more kinds selected from a dimer acid ester and an N-acyl amino acid ester in addition to components (A) to (F), thereby further enhancing water occlusive of a film obtained by application and further improving skin protective feeling and glossiness.

Examples of the dimer acid ester include an ester of a dimer acid and an alcohol, preferably an ester of a dimer dilinoleic acid, and more preferably an ester of a dimer dilinoleic acid and dimer diol. The ester moiety of a dimer acid or a dimer dilinoleic acid preferably contains one or more moieties selected from behenyl, isostearyl, stearyl, cetyl, and phytosteryl, and more preferably contains two or more moieties selected from behenyl, isostearyl, stearyl, cetyl, and phytosteryl. More specifically, dimer dilinoleic acid dimer dilinoleyl bis (behenyl/isostearyl/phytosteryl), dimer dilinoleic acid dimer dilinoleyl bis (phytosteryl/isostearyl/cetyl/stearyl/behenyl) , dimer dilinoleic acid (phytosteryl/isostearyl/cetyl/stearyl/behenyl) , dimer dilinoleic acid di(isostearyl/phytosteryl), and dimer dilinoleyl diisosteate, preferably dimer dilinoleic acid (phytosteryl/isostearyl/cetyl/stearyl/behenyl) and dimer dilinoleic acid di(isostearyl/phytosteryl), and more preferably dimer dilinoleic acid (phytosteryl/isostearyl/cetyl/stearyl/behenyl) .

Commercially available products thereof include "Plandool-S,-H" for dimer dilinoleic acid (phytosteryl/isostearyl/cetyl/stearyl/behenyl) ; "LUSPLAN PI-DA" for dimer dilinoleic acid di (isostearyl/phytosteryl); "LUSPLAN DD-DA" for dimer dilinoleic acid dimer dilinoleyl; "Plandool-G" for dimer dilinoleic acid dilinoleyl (behenyl/isostearyl/phytosteryl); and "LUSPLAN DD-IS" for dimer dilinoleyl diisostearate, all manufactured by Nippon Fine Chemical Co., Ltd.

The number of carbon atoms of the acyl group in the N-acyl amino acid ester is preferably from 10 to 30, more preferably from 12 to 18. The acyl group may be saturated or unsaturated. Example thereof include preferably a 2-ethylhexanoyl group, a lauroyl group, a myristoyl group, a palmitoyl group, a stearoyl group, a behenoyl group, an oleoyl group, an isostearoyl group, a linolenoyl group, or the like, more preferably a myristoyl group, or a lauroyl group, and

even more preferably a lauroyl group. The acyl group may be derived from a mixed fatty acid, and preferably is a cocoyl fatty acid ester, a palm oil fatty acid ester, a palm kernel oil fatty acid ester, a sunflower seed oil fatty acid ester, or a macadamia nut oil fatty acid ester, and more preferably is a cocoyl fatty acid ester or a palm kernel oil fatty acid ester.

The N-acyl amino acid ester is preferably an alkyl ester of an N-acyl amino acid. The number of carbon atoms of the alkyl group constituting the alkyl ester is preferably from 1 to 30, more preferably from 12 to 18. The alkyl group may be branched, linear, or cyclic. Examples thereof include octyl, lauryl, cetyl, stearyl, isostearyl, behenyl, octyldodecyl, phytosteryl such as campesteryl, or sitosteryl, cholesteryl, and decyl tetradecyl.

The amino acid moiety in the N-acyl amino acid ester is an acidic amino acid, a neutral amino acid, and a basic amino acid, preferably an acidic amino acid, and a neutral amino acid, and more preferably an acidic amino acid. Specific examples thereof include glutamic acid, aspartic acid, alanine, arginine, glycine, N-methylalanine, histidine, serine, threonine, sarcosine, and the like, preferably glutamic acid, aspartic acid, alanine, N-methylalanine, and sarcosine and more preferably glutamic acid.

It is also preferred that the N-acyl amino acid ester is a diester rather than a monoester. An N-acyl lauroyl glutamic acid diester, and an N-acyl aspartic acid diester are more preferred, and an N-acyl lauroyl glutamic acid diester is more preferred. The alkyl groups present in one molecule of diester may be the same or different, but two or more are preferably present.

In the present invention, the N-acyl amino acid esters may be contained singly or in a combination of two or more of them. For example, suitable examples thereof include di (cholesteryl/behenyl/octyldodecyl) N-lauroyl-L-glutamate, di (cholesteryl/octyldodecyl) N-lauroyl-L-glutamate, di (phytosteryl/behenyl/octyldodecyl) N-lauroyl-L-glutamate, di (phytosteryl/octyldodecyl) N-lauroyl-L-glutamate, and (phytosteryl/decyltetradecyl) N-myristoyl-N-methylalaninate, more suitable examples thereof include di(cholesteryl/octyldodecyl) N-lauroyl-L-glutamate and di(phytosteryl/octyldodecyl) N-lauroyl-L-glutamate.

Commercially available products of the N-acyl amino acid ester include "ELDEW PS-203" and "Plandool-LG2 (di(phytosteryl/octyldecyl) N-lauroyl-L-glutamate), "ELDEW CL-301 (di(cholesteryl/behenyl/octyldodecyl) N-lauroyl glutamate), "ELDEW CL-202 (di(cholesteryl/octyldodecyl) N-lauroyl glutamate), "ELDEW PS-304", "ELDEW PS-306", "Plandool-LG1", "Plandool-LG3" and "Plandool-LG4 (di(phytosteryl/ behenyl/octyldodecyl) N-lauroyl glutamate), "ELDEW APS-307" ((phytosteryl/decyltetradecyl) N-myristoyl-N-methylalaninate), and the like distributed by Ajinomoto Co., Inc. and Nippon Fine Chemical Co., Ltd.

These dimer acid ester and N-acyl-amino acid ester may be contained singly or in combination of two or more of them, and it is preferable to be contained in combination of two or more of them.

Component (G) is preferably one or more selected from a dimer acid ester and an N-acyl amino acid ester, more preferably one or more selected from a dimer dilinoleic acid ester and an N-acyl amino acid diester, and more preferably one or more selected from a dimer dilinoleic acid ester and an N-acyl glutamic acid diester. Further, it is preferable that component (G) containing a dimer acid ester and an N-acyl amino acid ester contains two or more kinds, and it is more preferable that component (G) contains both a dimer dilinoleic acid ester and an N-acylated glutamic acid diester.

From the viewpoint of enhancing water occlusive, skin protective feeling and glossiness and viewpoint of long-term storage stability, the content of component (G) is preferably 0.5% by mass or more, more preferably 1% by mass or more, even more preferably 2% by mass or more, even more preferably 2.5% by mass or more, and even more preferably 3% by mass or more; and preferably 20% by mass or less, more preferably 12% by mass or less, even more preferably 9 %by mass or less, and even more preferably 7% by mass or less in the water-in-oil emulsified composition of the present invention. The specific range is preferably 0.5% by mass or more and 20% by mass or less, more preferably 0.5% by mass or more and 12% by mass or less, even more preferably 1% by mass or more and 9% by mass or less, even more preferably 2% by mass or more and 9% by mass or less, even more preferably 2.5% by mass or more and 9% by mass or less, and even more preferably 3% by mass or more and 7% by mass or less.

In addition to the above components, the water-in-oil emulsion composition of the present invention may contain a liquid oil other than component (E), a humectant, a medicinal component, a coloring agent, a preservative, an antioxidant, a chelating agent, a perfume, and the like.

Here, examples of the liquid oil other than component (E) include silicone oil, ester oil, fluorine oil, and the like. Examples of humectant include a polyhydric alcohol such as glycerin, 1,3-butylene glycol, propylene glycol, 1,3-propanediol, dipropylene glycol, sorbitol, maltitol, mannitol, erythritol, xylitol, and trehalose, a plant extract, a betaine, POE methyl glucoside, and polyethylene glycol. As the medicinal component, either a water-soluble drug or a water-insoluble drug can be used, but specific examples thereof include allantoin, glycyrrhizic acid salt, glycyrrhizic acid, glycyrrhetinic acid ester such as stearyl glycyrrhetinate, isopropylmethylphenol, a retinol derivative, an anti-wrinkling agent, a whitening agent, a fungicide, a plant extract, tocopherol acetate, a vitamin, salicylic acid, indomethacin, and the like; and other medicinal component used in a cosmetic, a quasi-drug, and a pharmaceutical can be used also.

The water-in-oil emulsion composition of the present invention may be water-in-oil type and in a form that can be easily applied to the skin, and the viscosity thereof at 25°C is preferably 15 Pa·s or more, more preferably 20 Pa·s

or more, even more preferably 25 Pa·s or more, and even more preferably 30 Pa·s or more; and preferably 5000 Pa·s or less, more preferably 1000 Pa·s or less, even more preferably 500 Pa·s or less, even more preferably 300 Pa·s or less, and even more preferably 150 Pa·s or less. The specific range is preferably 15 Pa·s or more and 5000 Pa·s or less resulting in cream form, more preferably 20 Pa·s or more and 1000 Pa·s or less resulting in cream form, even more preferably 20 Pa·s or more and 500 Pa·s or less resulting in a cream form, even more preferably 25 Pa·s or more and 300 Pa·s or less resulting in a cream form, and even more preferably 30 Pa·s or more and 150 Pa·s or less resulting in a cream form. Here, the viscosity at 25°C can be measured by using a B8R type viscometer, under a condition of 1 minute and 25°C.

In the water-in-oil emulsion composition of the present invention, for example, oily components (water-insoluble components) are mixed by heating and stirring, and then water-soluble components are mixed by heating and stirring according to a conventional method. The water-oil emulsion composition can be prepared by adding the mixture of the water-soluble components to the mixture of the oily components, mixing the resulting mixture, stirring the mixture thoroughly with a homogenizer, and then cooling the mixture.

The use of the water-in-oil emulsion composition of the present invention is not particularly limited, but is preferably application to the hair and/or skin, and is more preferably application to the skin of face, body, limb, and the like. It is also preferably used as a skin external preparation, and more preferably as a skin cosmetic. The method of use is not particularly limited, but it is preferably applied to the hair and/or skin by hand or tool, and more preferably to the skin by hand or tool.

With respect to the above-described embodiments, the present invention further discloses the following compositions.

<1> A water-in-oil emulsion composition comprising the following components (A), (B), (C), (D), (E) and (F):

(A) 1% by mass or more and 30% by mass or less of an oily component having a melting point of from 50 to 150°C;
(B) 0.5% by mass or more and 8% by mass or less of a polyglycerol fatty acid ester;
(C) a lipophilic surfactant other than component (B), having an HLB of from 2 to 6;
(D) 0.15% by mass or more and 8% by mass or less of a dextrin fatty acid ester having 8 to 22 carbon atoms in the fatty acid moiety;
(E) a hydrocarbon oil that is liquid at 25°C; and
(F) water.

<2> The water-in-oil emulsion composition according to <1>, wherein component (A) is one or more selected from the group consisting of a sphingolipid such as a ceramide and a sphingosines (including natural and synthetic products); a sterol and an analogue thereof such as cholesterol, dehydrocholesterol, β-sitosterol, stearoyl cholesteryl ester, isostearoyl cholesteryl ester, and vegetable oil fatty acid cholesteryl ester; a $C_{16}$-$C_{22}$ fatty acid such as stearic acid and behenic acid; and a $C_{16}$-$C_{22}$ alcohol and an analogue thereof such as cetyl alcohol, stearyl alcohol, behenyl alcohol, batyl alcohol, and kimyl alcohol; preferably one or more selected from the group consisting of a ceramide (in particular, a natural ceramide, a sphingosine, a compound of the above formulae (1) and (2)), a $C_{16}$-$C_{22}$ fatty acid, and a $C_{16}$-$C_{22}$ alcohol; more preferably one or more selected from the group consisting of compounds of the above formulae (1) and (2); even more preferably a compound of the above formula (1).

<3> The water-in-oil emulsion composition according to <1> or <2>, wherein the content of component (A) is 1.5% by mass or more, more preferably 2% by mass or more, even more preferably 3% by mass or more, and even more preferably 5% by mass or more; and preferably 20% by mass or less, more preferably 18% by mass or less, and even more preferably 16% by mass or less in the water-in-oil emulsion composition.

<4> The water-in-oil emulsion composition according to any one of <1> to <3>, wherein component (B) is preferably a polyglycerol difatty acid ester and a polyglycerol trifatty acid ester; more preferably a polyglycerol di $C_{16}$-$C_{22}$ fatty acid ester and a polyglycerol tri $C_{16}$-$C_{22}$ fatty acid ester; even more preferably polyglycerol diisostearate and polyglycerol triisostearate.

<5> The water-in-oil emulsion composition according to any one of <1> to <4>, wherein the content of component (B) is preferably 0.7% by mass or more, more preferably 0.8% by mass or more, and even more preferably 1% by mass or more; and preferably 7% by mass or less, even more preferably 6% by mass or less, even more preferably 5% by mass or less, and even more preferably 3% by mass or less in the water-in-oil emulsion composition.

<6> The water-in-oil emulsion composition according to any one of <1> to <5>, wherein component (C) is preferably a nonionic surfactant having an HLB in the range of from 2 to 6, preferably one or more selected from the group consisting of glycerol fatty acid ester, a propylene glycol fatty acid ester, a sorbitan fatty acid ester, a polyoxyethylene castor oil, a polyoxyethylene hydrogenated castor oil, a polyoxyethylene hydrogenated castor oil fatty acid ester, a polyethylene glycol fatty acid ester, an alkyl glyceryl ether, an alkyl polyglyceryl ether, a polyoxyethylene alkyl ether, a polyoxyethylene alkyl ether fatty acid ester, a polyoxyethylene alkyl amine, and a silicone surfactant; more preferably

one or more selected from the group consisting of a polyoxyethylene hydrogenated castor oil, an alkyl glyceryl ether, a polyether modified silicone having a linear silicone chain, and a polyether modified silicone having a branched silicone chain; even more preferably an alkyl glyceryl ether and a polyether modified silicone having a linear silicone chain.

<7> The water-in-oil emulsion composition according to any one of <1> to <6>, wherein the content of component (C) is preferably 0.5% by mass or more, more preferably 0.8% by mass or more, and even more preferably 1% by mass or more; and preferably 8% by mass or less, more preferably 7% by mass or less, even more preferably 5% by mass or less, even more preferably 3.5% by mass or less, and even more preferably 2% by mass or less in the water-in-oil emulsion composition.

<8> The water-in-oil emulsion composition according to any one of <1> to <7>, wherein the total content of components (B) and (C) is 1.0% by mass or more, more preferably 1.5% by mass or more, more preferably 2% by mass or more, and even more preferably 2.5% by mass or more; and preferably 12% by mass or less, more preferably 10% by mass or less, even more preferably 8% by mass or less, even more preferably 6% by mass or less, even more preferably 5% by mass or less, and even more preferably 4.5% by mass or less in the water-in-oil emulsion composition.

<9> The water-in-oil emulsion composition according to any one of <1> to <8>, wherein component (D) is preferably a dextrin fatty acid ester having 12 to 18 carbon atoms in the fatty acid moiety, more preferably a dextrin fatty acid ester having 14 to 18 carbon atoms in the fatty acid moiety, even more preferably a dextrin fatty acid ester having 14 to 16 carbon atoms in the fatty acid moiety, and even more preferably a dextrin fatty acid ester having 16 carbon atoms in the fatty acid moiety.

<10> The water-in-oil emulsion composition according to any one of <1> to <9>, wherein the content of component (D) is preferably 0.2% by mass or more, more preferably 0.3% by mass or more, and even more preferably 0.4% by mass or more; and preferably 6% by mass or less, even more preferably 5% by mass or less, even more preferably 4% by mass or less, and even more preferably 2% by mass or less in the water-in-oil emulsion composition.

<11> The water-i.n-oil emulsion composition according to any one of <1> to <10>, wherein component (E) is a linear or branched hydrocarbon oil such as liquid paraffin, liquid isoparaffin, squalane, and squalene, preferably squalane.

<12> The water-in-oil emulsion composition according to any one of <1 >to <11>, wherein the content of component (E) is preferably 3% by mass or more, more preferably 4% by mass or more, even more preferably 5% by mass or more, and even more preferably 7% by mass or more; preferably 20% by mass or less, more preferably 18% by mass or less, even more preferably 16% by mass or less, and even more preferably 12% by mass or less in the water-in-oil emulsion composition.

<13> The water-in-oil emulsion composition according to any one of <1> to <12>, wherein the content of component (F) is 10% by mass or more, more preferably 15% by mass or more, and even more preferably 20% by mass or more; and preferably 65% by mass or less, more preferably 60% by mass or less, and even more preferably 50% by mass or less.

<14> The water-in-oil emulsion composition according to any one of <1> to <13>, which further comprises (G) one or more selected from the group consisting of a dimer acid ester and an N-acyl amino acid ester.

<15> The water-in-oil emulsion composition according to <14>, wherein component (G) is preferably one or more selected from the group consisting of a dimer dilinoleic acid ester and an N-acyl amino acid diester, and more preferably one or more selected from the group consisting of a dimer dilinoleic acid ester and an N-acyl glutamic acid diester.

<16> The water-in-oil emulsion composition according to <14> or <15>, wherein the content of component (G) is preferably 0.5% by mass or more, more preferably 1% by mass or more, even more preferably 2% by mass or more, and even more preferably 3% by mass or more; and preferably 12% by mass or less, more preferably 9% by mass or less, and even more preferably 7% by mass or less.

<17> The water-in-oil emulsion composition according to any one of <1> to <16>, wherein the viscosity thereof at 25°C is preferably 15 Pa·s or more, more preferably 20 Pa·s or more, even more preferably 25 Pa·s or more, and even more preferably 30 Pa·s or more; and preferably 5000 Pa·s or less, more preferably 1000 Pa·s or less, even more preferably 500 Pa·s or less, even more preferably 300 Pa·s or less, and even more preferably 150 Pa·s or less..

<18> A water-in-oil emulsion composition comprising the following components (A), (B), (C), (D), (E) and (F) :

(A) 1% by mass or more and 30% by mass or less of a ceramide;
(B) 0.5% by mass or more and 8% by mass or less of a polyglycerol fatty acid ester;
(C) a polyether modified silicone having an HLB of from 2 to 6;
(D) 0.15% by mass or more and 8% by mass or less of a dextrin fatty acid ester having 12 to 18 carbon atoms in the fatty acid moiety;
(E) a hydrocarbon oil that is liquid at 25°C; and
(F) water.

<19> The water-in-oil emulsion composition according to <18>, wherein component (B) is one or more selected from the group consisting of a polyglycerol difatty acid ester and a polyglycerol trifatty acid ester.
<20> The water-in-oil emulsion composition according to <18> or <19>, wherein component (A) is a compound of the formula (1).
<21> The water-in-oil emulsion composition according to any one of <18> to <20>, wherein component (E) is a squalane.
<22> The water-in-oil emulsion composition according to any one of <18> to <21>, further comprising component (G) which is one or more selected from the group consisting of a dimer acid ester and an N-acyl amino acid ester.
<23> The water-in-oil emulsion composition according to any one of <1> to <22>, which is a skin external preparation, preferably a skin cosmetic.
<24> A method of using a water-in-oil emulsion composition, comprising: applying the water-in-oil emulsion composition according to any <1> to <23> to the skin excluding hair, preferably any of the face, body, or limb.

## EXAMPLES

Next, the present invention will be described in more detail with reference to Examples and Comparative Examples.

### Examples 1 to 21 and Comparative Examples 1 to 7

Water-in-oil emulsion compositions were prepared using the components shown in Tables 1 to 6. That is, the oily components (water-insoluble components) were stirred and mixed at from 80 to 85°C, and then the water-soluble components were stirred and mixed at from 80 to 85°C. The mixture of the above water-soluble components was added to the mixture of the above oily components and mixed, and thoroughly stirred with a homogenizer at from 80 to 85°C, followed by cooling to obtain a water-in-oil emulsion composition.
The resulting water-in-oil emulsion composition was evaluated for water occlusive (water evaporation suppression rate), skin protective feeling (skin-covering feeling), skin glossiness (glossy skin), and a feeling of the applied skin without stickiness and uncomfortable feeling according to the following evaluation method. Storage stability was also evaluated.
The evaluation results are shown in Tables 1 to 6.

### (1) Water evaporation suppression rate

- Filter paper (No. 5B, 21-mm diameter, manufactured by ADVANTEC Corporation)
- Pierce Vial CV-400 (product distributed by AS ONE Corporation, product code 5-106-06, inner diameter × barrel diameter × overall height: φ17 × φ27 × 95 mm, volume: 40 mL)
- Calcium chloride (for drying): manufactured by Junsei Chemical Co., Ltd.
- Desiccator
- Microman (Gilson Incorporated.)

(i) The resin lid attached to the screw cap of the pierce vial was removed.
(ii) The prepared water-in-oil emulsion composition was placed in an amount of 0.03 mL using Microman on a filter paper, and uniformly applied to one side of the filter paper with a spatula.
(iii) The filter paper was placed in the screw cap such that the surface applied with the water-in-oil emulsion composition was contacted with the inner surface of the screw cap of the Pierce Vial. A sample of only the filter paper to which the water-in-oil emulsion composition was not applied was also prepared.
(iv) The Pierce Vial was filled with 10 mL of distilled water, closed with the screw cap in which the filter paper prepared in (iii) was placed, and used as a sample.
(v) The weight of the sample (=total weight of preparation-applied filter paper, screw cap, vial, water) was measured with an electronic balance.
(vi) Calcium chloride (for drying) was placed in a desiccator, the sample was placed in the desiccator, a lid was put thereon, and the desiccator was left to stand at 20°C for 5 days. At this time, 5RH% was kept in the desiccator.
(vii) The sample was taken out of the desiccator and the weight of the sample (=total weight of formulation-applied filter paper, screw cap, vial, water) was measured.
(viii) Three samples were prepared for each of Examples and Comparative Examples. Three samples were also prepared for filter paper to which nothing was applied.

(ix) The water evaporation amount was measured, and the water evaporation suppression rate of the water-in-oil emulsion composition shown in Examples and Comparative Examples was calculated.

Water evaporation ($W_a$) of sample applied with water-in-oil emulsion composition = (sample weight after drying for 5 days) - (sample weight prior to drying)

$$W_a \text{ mean} = (W_{a1} + W_{a2} + W_{a3})/3$$

Water evaporation ($W_0$) of sample not applied with water-in-oil emulsion composition = (sample weight after drying for 5 days) - (sample weight prior to drying)

$$W_0 \text{ mean} = (W_{01} + W_{02} + W_{03})/3$$

Water evaporation suppression rate (%) = (($W_0$ mean - $W_a$ mean)/$W_0$ mean) × 100

(2) Protective feeling of the skin and glossiness of the skin

The water-in-oil emulsion compositions of Examples and Comparative Examples were evaluated according to the following methods of use by four expert panelists.

(i) Expert panelists washed his/her face with a commercially available Curel-moisturizing foam face wash manufactured by Kao Corporation and wiped it with a towel.
(ii) The water-in-oil emulsion composition was taken with fingers in an amount of 0.05 g and spread over the entire face.
(iii) Ten minutes after application, the expert panelists evaluated the skin protective feeling on the basis of the following criteria, and the average score of four panelists was obtained. In addition, expert panelists looked at the mirror in a room with a constant brightness to evaluate the glossiness of the skin on the basis of the following criteria, and the average score of four panelists was obtained.

(Skin protective feeling)

5: The skin feels very covered.
4: Felt the skin covered.
3: Slightly felt the skin covered.
2: Hardly felt the skin covered.
1: Not felt the skin covered at all.

(Skin glossiness)

5: Very glossy.
4: Glossy.
3: Slightly glossy.
2: Less glossy.
1: Not glossy.

(3) No stickiness and no uncomfortable feeling

The water-in-oil emulsion compositions of Examples and Comparative Examples were evaluated according to the following methods of use by four expert panelists.

(i) Expert panelists washed his/her face with a commercially available Curel-moisturizing foam face wash manufactured by Kao Corporation and wiped it with a towel.
(ii) The water-in-oil emulsion composition was taken with fingers in an amount of 0.05 g and spread over the entire face.
(iii) Three hours after application, expert panelists evaluated the feeling of burden on the skin on the basis of the following criteria, and the average score of four panelists was obtained.

5: No stickiness and no uncomfortable feeling.
4: Almost no stickiness and no uncomfortable feeling.
3: Not much stickiness and not much uncomfortable feeling.
2: Sticky and slight uncomfortable feeling.
1: Very sticky and uncomfortable feeling.

(3) Storage stability

The water-in-oil emulsion compositions of the Examples and Comparative Examples were placed in a 110 mL transparent glass container (standard bottle #11, Tokyo Garasu Kikai Co., Ltd.) in a volume of 80 mL, sealed, and stored at room temperature for a certain period of time. The appearance of these water-in-oil emulsion compositions after storage was visually determined on the basis of the following criteria.

A: A uniform single layer and not observed separation of aqueous or oily components. Creamy.
B: Observed partial separation of aqueous and oily components, but maintained creamy as a whole.
C: Entire separation of aqueous and the oily components and not maintained creamy as a whole.

(4) Viscosity

Using a TVB-10 viscometer manufactured by Toki Sangyo Co., Ltd., the viscosity was measured under the condition of rotors: T-C, rotational speeds: 5 rpm, measurement time: 1 minute, and measurement temperature: 25°C.

Table 1

| | | Example: | | | | | Comparative Example |
|---|---|---|---|---|---|---|---|
| Component | Component name | 1 | 2 | 3 | 4 | 5 | 1 |
| | Allantoin | 0.5 | 0.5 | 0.5 | 0.5 | 0.5 | 0.5 |
| (A) | N-(hexadecyloxyhydroxypropyl)-N-hydroxyethylhexadecanamide *1 | 8.0 | 3.0 | | | 15.0 | |
| | Stearic acid | | | | 6.0 | | |
| | Stearyl alcohol | | | 6.0 | | | |

(continued)

| | | Example: | | | | | Comparative Example |
|---|---|---|---|---|---|---|---|
| Component | Component name | 1 | 2 | 3 | 4 | 5 | 1 |
| (C) | Isostearyl glyceryl ether *2 | 0.2 | 0.2 | 0.2 | 0.2 | 0.2 | 0.2 |
| | Polyoxyethylene/methylpolysiloxane copolymer *3 | 1.5 | 1.5 | 1.5 | 1.5 | 1.5 | 1.5 |
| (B) | Polyglyceryl diisostearate *4 | 2.0 | 2.0 | 2.0 | 2.0 | 2.0 | 2.0 |
| (E) | Squalane | 8.0 | 8.0 | 8.0 | 8.0 | 8.0 | 8.0 |
| | Methylpolysiloxane *5 | 8.0 | 8.0 | 8.0 | 8.0 | 8.0 | 8.0 |
| | Methylpolysiloxane *6 | 4.0 | 4.0 | 4.0 | 4.0 | 4.0 | 4.0 |
| | Methylpolysiloxane-crosslinked methylpolysiloxane mixture *7 | 5.0 | 5.0 | 5.0 | 5.0 | 5.0 | 5.0 |
| (G) | Di(cholesteryl/octyldodecyl) N-lauroyl-L-glutamate *8 | 1.0 | 1.0 | 1.0 | 1.0 | 1.0 | 1.0 |
| | Di(phytosteryl/2-octyldodecyl) N-lauroyl-L-glutamate *9 | 2.0 | 2.0 | 2.0 | 2.0 | 2.0 | 2.0 |
| | Di(phytosteryl/isostearyl/cetyl/stearyl/behenyl) dilinolate *10 | 2.5 | 2.5 | 2.5 | 2.5 | 2.5 | 2.5 |
| (D) | Dextrin palmitate *11 | 0.5 | 0.5 | 0.5 | 0.5 | 0.5 | 0.5 |
| | Magnesium sulfate heptahydrate | 0.7 | 0.7 | 0.7 | 0.7 | 0.7 | 0.7 |
| | Glycerin (86%) | 10.0 | 10.0 | 10.0 | 10.0 | 10.0 | 10.0 |
| | 1,3-Butylene glycol | 4.0 | 4.0 | 4.0 | 4.0 | 4.0 | 4.0 |
| | Eucalyptus extract | 0.33 | 0.33 | 0.33 | 0.33 | 0.33 | 0.33 |
| | Water-soluble ginger extract (K) | 0.5 | 0.5 | 0.5 | 0.5 | 0.5 | 0.5 |
| | Hiba arborvitae extract | 2.0 | 2.0 | 2.0 | 2.0 | 2.0 | 2.0 |
| | Methylparaben | 0.33 | 0.33 | 0.33 | 0.33 | 0.33 | 0.33 |
| | Succinic acid | 0.1 | 0.1 | 0.1 | 0.1 | 0.1 | 0.1 |
| | Sodium hydroxide solution (48%) | 0.07 | 0.07 | 0.07 | 0.07 | 0.07 | 0.07 |
| (E) | Water | 38.77 | 43.77 | 40.77 | 40.77 | 31.77 | 46.77 |
| | Total | 100.0 | 100.0 | 100.0 | 100.0 | 100.0 | 100.0 |
| | Component (A) | 8.0 | 3.0 | 6.0 | 6.0 | 15.0 | 0.0 |
| | Component (B) | 2.0 | 2.0 | 2.0 | 2.0 | 2.0 | 2.0 |
| | Component (C) | 1.7 | 1.7 | 1.7 | 1.7 | 1.7 | 1.7 |
| | Component (D) | 0.5 | 0.5 | 0.5 | 0.5 | 0.5 | 0.5 |
| | Component (E) | 8.0 | 8.0 | 8.0 | 8.0 | 8.0 | 8.0 |
| | Component (F) | 38.77 | 43.77 | 40.77 | 40.77 | 31.77 | 46.77 |
| | Viscosity (Pa·s) | 48 | 45 | 38 | 40 | 120 | 35 |

(continued)

| | | Example: | | | | | Comparative Example |
|---|---|---|---|---|---|---|---|
| Component | Component name | 1 | 2 | 3 | 4 | 5 | 1 |
| Evaluation | Water evaporation suppression rate (%) | 49 | 27 | 18 | 12 | 52 | 7 |
| | Skin-covering feeling | 5.0 | 3.5 | 3.0 | 2.8 | 5.0 | 2.0 |
| | Glossiness of skin | 5.0 | 5.0 | 5.0 | 5.0 | 5.0 | 4.5 |
| | No stickiness and no uncomfortable feeling | 5.0 | 5.0 | 2.8 | 2.5 | 5.0 | 3.5 |
| | Storage stability (room temperature, 1 day) | A | A | A | A | A | C |
| | Storage stability (room temperature, 1 month) | A | A | A | A | A | C |

*1: Sphingolipid E (Kao Corporation)
*2: GE-IS (U) (HLB 2) (Kao Corporation)
*3: Silicone KF-6015 (PEG-3 dimethicone, HLB 4.5 (Shin-Etsu Chemical Co., Ltd.)
*4: Cosmol 42V (polyglyceryl-2 diisostearate) (Nisshin Oilio Group Ltd.)
*5: Silicone KF-96A-2CS (Shin-Etsu Chemical Co., Ltd.)
*6: Silicone KF-96A-6CS (Shin-Etsu Chemical Co., Ltd.)
*7: Silicone KSG-16 (Shin-Etsu Chemical Co., Ltd.)
*8: ELDEW CL-202 (Ajinomoto Co., Inc.)
*9: ELDEW PS-203 (Ajinomoto Co., Inc.)
*10: Plandool-H (manufactured by Nippon Fine Chemical Co., Ltd.)
*11: Rheopearl KL2 (Chiba Flour Milling Co., Ltd.)

Table 2

| | | Example: | | | Comparative Example |
|---|---|---|---|---|---|
| Component | Component name | 6 | 7 | 8 | 2 |
| | Allantoin | 0.5 | 0.5 | 0.5 | 0.5 |
| (A) | N-(hexadecyloxyhydroxypropyl)-N-hydroxyethylhexadecanamide *1 | 8.0 | 8.0 | 8.0 | 8.0 |
| (C) | Isostearyl glyceryl ether *2 | 0.2 | 0.2 | 0.2 | 0.2 |
| | Polyoxyethylene/methylpolysiloxane copolymer *3 | 1.5 | 1.5 | 1.5 | 1.5 |
| (B) | Polyglyceryl diisostearate *4 | 1.0 | 5.0 | | |
| | Polyglyceryl triisostearate *12 | | | 2.0 | |
| (E) | Squalane | 10.0 | 10.0 | 8.0 | 8.0 |
| | Methylpolysiloxane *5 | | | 8.0 | 8.0 |
| | Methylpolysiloxane *6 | 6.0 | 4.0 | 4.0 | 4.0 |
| | Methylpolysiloxane-crosslinked methylpolysiloxane mixture *7 | 5.0 | 5.0 | 5.0 | 5.0 |
| (G) | Di(cholesteryl/octyldodecyl) N-lauroyl-L-glutamate *8 | 1.0 | | 1.0 | |
| | Di(phytosteryl/2-octyldodecyl) N-lauroyl-L-glutamate *9 | 2.0 | | 2.0 | |
| | Di(phytosteryl/isostearyl/cetyl/stearyl/behenyl) dilinolate *10 | 2.5 | 2.5 | 2.5 | |
| (D) | Dextrin palmitate *11 | 0.5 | 0.5 | 0.5 | 0.5 |

(continued)

| | | Example: | | | Comparative Example |
|---|---|---|---|---|---|
| Component | Component name | 6 | 7 | 8 | 2 |
| | Magnesium sulfate heptahydrate | 0.7 | 0.7 | 0.7 | 0.7 |
| | Glycerin (86%) | 10.0 | 10.0 | 10.0 | 10.0 |
| | Eucalyptus extract | 0.33 | 0.33 | 0.33 | 0.33 |
| | Water-soluble ginger extract (K) | 0.5 | 0.5 | 0.5 | 0.5 |
| | Hiba arborvitae extract | 2.0 | 2.0 | 2.0 | 2.0 |
| | 1,3-Butylene glycol | 4.0 | 4.0 | 4.0 | 4.0 |
| | Methylparaben | 0.33 | 0.33 | 0.33 | 0.33 |
| | Succinic acid | 0.1 | 0.1 | 0.1 | 0.1 |
| | Sodium hydroxide solution (48%) | 0.07 | 0.07 | 0.07 | 0.07 |
| (E) | Water | 43.77 | 44.77 | 38.77 | 46.27 |
| | Total | 100.0 | 100.0 | 100.0 | 100.0 |
| | Component (A) | 8.0 | 8.0 | 8.0 | 8.00 |
| | Component (B) | 1.0 | 5.0 | 2.0 | 0.0 |
| | Component (C) | 1.7 | 1.7 | 1.7 | 1.7 |
| | Component (D) | 0.5 | 0.5 | 0.5 | 0.5 |
| | Component (E) | 10.0 | 10.0 | 8.0 | 8.0 |
| | Component (F) | 43.77 | 44.77 | 38.77 | 46.27 |
| Evaluation | Viscosity (Pa·s) | 64 | 64 | 48 | 86 |
| | Water evaporation suppression rate (%) | 45 | 39 | 45 | 33 |
| | Skin-covering feeling | 4.8 | 4.3 | 5.0 | 4.0 |
| | Glossiness of skin | 5.0 | 4.3 | 5.0 | 1.3 |
| | No stickiness and no uncomfortable feeling | 5.0 | 3.8 | 4.3 | 5.0 |
| | Storage stability (room temperature, 1 day) | A | A | A | A |
| | Storage stability (room temperature, 1 month) | A | A | A | A |

*1: Sphingolipid E (Kao Corporation)
*2: GE-IS (U) (HLB 2) (Kao Corporation)
*3: Silicone KF-6015 (PEG-3 dimethicone, HLB 4.5 (Shin-Etsu Chemical Co., Ltd.)
*4: Cosmol 42V (polyglyceryl-2 diisostearate) (Nisshin Oilio Group Ltd.)
*5: Silicone KF-96A-2CS (Shin-Etsu Chemical Co., Ltd.)
*6: Silicone KF-96A-6CS (Shin-Etsu Chemical Co., Ltd.)
*7: Silicone KSG-16 (Shin-Etsu Chemical Co., Ltd.)
*8: ELDEW CL-202 (Ajinomoto Co., Inc.)
*9: ELDEW PS-203 (Ajinomoto Co., Inc.)
*10: Plandool-H (Nippon Fine Chemical Co., Ltd.)
*11: Rheopearl KL2 (Chiba Flour Milling Co., Ltd.)
*12: Cosmol 43V (Nisshin Oilio Group Ltd.)

Table 3

| | | Example | | | | Comparative Example | |
|---|---|---|---|---|---|---|---|
| Component | Component name | 9 | 10 | 11 | 12 | 3 | 4 |
| | Allantoin | 0.5 | 0.5 | 0.5 | 0.5 | 0.5 | 0.5 |
| (A) | N-(hexadecyloxyhydroxypropyl)-N-hydroxyethylhexadecanamide *1 | 8.0 | 3.0 | 8.0 | 8.0 | 8.0 | 8.0 |
| | Long-chain dibasic acid bis-3-methoxypropylamide *13 | | | | | 1.0 | |
| (C) | Isostearyl glyceryl ether *2 | 0.2 | 0.2 | 0.2 | 0.2 | 2.0 | 0.2 |
| | Polyoxyethylene/ methylpolysiloxane copolymer *14 | | | | | 0.2 | 0.2 |
| | Polyoxyethylene/ methylpolysiloxane copolymer *3 | 1.5 | 1.5 | 1.5 | 1.5 | | 1.5 |
| | Polyoxyethylene hydrogenated castor oil *15 | | | | | 0.5 | |
| (B) | Polyglyceryl diidostearate *4 | 2.0 | 2.0 | 2.0 | 2.0 | 2.0 | |
| (E) | Squalane | 8.0 | 8.0 | 8.0 | 8.0 | 3.5 | 6.0 |
| | Methylcyclopolysiloxane *16 | | | | | 8.0 | 8.0 |
| | Octamethyltrisiloxane *17 | | | | | 3.5 | 5.0 |
| | Methylpolysiloxane *5 | 8.0 | 8.0 | 8.0 | 8.0 | | |
| | Methylpolysiloxane *6 | 4.0 | 4.0 | 4.0 | 4.0 | 2.0 | 2.0 |
| | Methylpolysiloxane-crosslink methylpolysiloxane mixture *7 | 5.0 | 5.0 | 5.0 | 5.0 | | |
| | Dimethyl palmityl polysiloxane*18 | | | | | 0.5 | |
| | Cholesteryl isostearate *19 | | | | | 1.0 | |
| (G) | Di(phytosteryl/isostearyl/cetyl/ stearyl/behenyl) dilinolate *10 | 2.5 | 2.5 | 2.5 | 2.5 | | |
| | Di(cholesteryl/octyldodecyl) N-lauroyl-L-glutamate *8 | 1.0 | 1.0 | 1.0 | 1.0 | | |
| | Di(phytosteryl/2-octyldodecyl) N-lauroyl-L-glutamate *9 | 2.0 | 2.0 | 2.0 | 2.0 | | |
| (D) | Dextrin palmitate *11 | 1.5 | 4.0 | | 0.25 | 0.1 | |
| | Dextrin myristate *20 | | | 0.5 | | | |
| | Magnesium sulfate heptahydrate | 0.7 | 0.7 | 0.7 | 0.7 | 0.5 | 0.3 |
| | Glycerin (86%) | 10.0 | 10.0 | 10.0 | 10.0 | 15.0 | 15.0 |
| | Eucalyptus extract | | | | | 0.5 | 0.5 |
| | Eucalyptus extract | 0.33 | 0.33 | 0.33 | 0.33 | | |
| | Water-soluble ginger extract (K) | 0.5 | 0.5 | 0.5 | 0.5 | | |
| | Hiba arborvitae extract | 2.0 | 2.0 | 2.0 | 2.0 | | |
| | 1,3-Butylene glycol | 4.0 | 4.0 | 4.0 | 4.0 | | |
| | Methylparaben | 0.33 | 0.33 | 0.33 | 0.33 | 0.3 | 0.2 |
| | Succinic acid | 0.1 | 0.1 | 0.1 | 0.1 | 0.24 | 0.1 |

(continued)

| | | Example | | | | Comparative Example | |
|---|---|---|---|---|---|---|---|
| Component | Component name | 9 | 10 | 11 | 12 | 3 | 4 |
| | Sodium hydroxide solution (48%) | 0.07 | 0.07 | 0.07 | 0.07 | 0.07 | 0.07 |
| (F) | Water | 37.77 | 40.27 | 38.77 | 39.02 | 50.59 | 52.43 |
| | Total | 100.0 | 100.0 | 100.0 | 100.0 | 100.0 | 100.0 |
| | Component (A) | 8.0 | 3.0 | 8.0 | 8.0 | 9.0 | 8.0 |
| | Component (B) | 2.0 | 2.0 | 2.0 | 2.0 | 2.0 | 2.0 |
| | Component (C) | 1.7 | 1.7 | 1.7 | 1.7 | 2.7 | 1.9 |
| | Component (D) | 1.5 | 4.0 | 0.5 | 0.25 | 0.1 | 0.0 |
| | Component (E) | 8.0 | 8.0 | 8.0 | 8.0 | 3.5 | 6.0 |
| | Component (F) | 37.77 | 40.27 | 38.77 | 39.02 | 50.59 | 52.43 |
| | Viscosity (Pa·s) | 84 | 50 | 56 | 45 | 85 | 50 |
| Evaluation | Water evaporation suppression rate (%) | 44 | 55 | 49 | 43 | 5 | 3 |
| | Skin-covering feeling | 4.8 | 5.0 | 5.0 | 4,5 | 2.0 | 1.3 |
| | Glossiness of skin | 5.0 | 5.0 | 5.0 | 5.0 | 2.5 | 1.3 |
| | No stickiness and no uncomfortable feeling | 3.8 | 2.8 | 4.8 | 5.0 | 5.0 | 5.0 |
| | Storage stability (room temperature, 1 day) | A | A | A | A | A | A |
| | Storage stability (room temperature, 1 month) | A | A | A | A | A | A |

*1: Sphingolipid E (Kao Corporation)
*2: GE-IS (U) (HLB 2) (Kao Corporation)
*3: Silicone KF-6015 (PEG-3 dimethicone, HLB 4.5 (Shin-Etsu Chemical Co., Ltd.)
*4: Cosmol 42V (polyglyceryl-2 diisostearate) (Nisshin Oilio Group Ltd.)
*5: Silicone KF-96A-2CS (Shin-Etsu Chemical Co., Ltd.)
*6: Silicone KF-96A-6CS (Shin-Etsu Chemical Co., Ltd.)
*7: Silicone KSG-16 (Shin-Etsu Chemical Co., Ltd.)
*8: ELDEW CL-202 (Ajinomoto Co., Inc.)
*9: ELDEW PS-203 (Ajinomoto Co., Inc.)
*10: Plandool-H (by Nippon Fine Chemical Co., Ltd.)
*11: Rheopearl KL2 (Chiba Flour Milling Co., Ltd.)
*13: BRS661-C (Kao Corporation)
*14: Silicone SH3775M (PEG-12 dimethicone, HLB 5 (Dow Corning Toray Co., Ltd)
*15: Emulex HC-5 (PEG-5 hydrogenated castor oil, HLB 5 (Nippon Emulsion Co., Ltd.)
*16: Silicone TSF 405 A (Momentive Performance Materials Japan Inc.)
*17: Silicone KF-96A-1CS (Shin-Etsu Chemical Co., Ltd.)
*18: Silicone KT-16 (Momentive Performance Materials Japan Inc.)
*19: Exepearl IS-CE-A (Kao Corporation)
*20: Rheopearl MKL2 (Chiba Flour Milling Co., Ltd.)

Table 4

| | | Example | | | |
|---|---|---|---|---|---|
| Component | Component name | 13 | 14 | 15 | 16 |
| | Allantoin | 0.5 | 0.5 | 0.5 | 0.5 |
| (A) | N-(hexadecyloxyhydroxypropyl)-N-hydroxyethylhexadecanamide *1 | 8.0 | 8.0 | 8.0 | 8.0 |
| (C) | Isostearyl glyceryl ether *2 | 0.2 | | | 0.2 |
| | Polyoxyethylene/methylpolysiloxane copolymer *3 | 1.5 | | | |
| | Polyoxyethylene/methylpolysiloxane copolymer *21 | | | | 0.75 |
| | PEG-9 polydimethylsiloxyethyl dimethicone*22 | | | 2.0 | |
| | Polyoxyethylene hydrogenated castor oil *15 | | 4.0 | | |
| (B) | Polyglyceryl diisostearate *4 | 2.0 | 2.0 | 2.0 | 2.0 |
| (E) | Squalane | 8.0 | 8.0 | 8.0 | 8.0 |
| | Methylpolysiloxane *5 | 8.0 | 8.0 | 8.0 | 8.0 |
| | Methylpolysiloxane *6 | 4.0 | 4.0 | 4.0 | 4.0 |
| | Methylpolysiloxane-crosslinked methylpolysiloxane mixture *7 | 5.0 | 5.0 | 5.0 | 5.0 |
| (G) | Di(cholesteryl/octyldodecyl) N-lauroyl-L-glutamate *8 | 1.0 | 1.0 | 1.0 | 1.0 |
| | Di(phytosteryl/2-octyldodecyl) N-lauroyl-L-glutamate *9 | 2.0 | 2.0 | 2.0 | 2.0 |
| | Di(phytosteryl/isostearyl/cetyl/stearyl/behenyl) dilinolate *10 | 2.5 | 2.5 | 2.5 | 2.5 |
| (D) | Dextrin palmitate *11 | 0.5 | 0.5 | 0.5 | 0.5 |
| | Magnesium sulfate heptahydrate | 0.7 | 0.7 | 0.7 | 0.7 |
| | Glycerin (86%) | 10.0 | 10.0 | 10.0 | 10.0 |
| | 1,3-Butylene glycol | 4.0 | 4.0 | 4.0 | 4.0 |
| | Eucalyptus extract | 0.33 | 0.33 | 0.33 | 0.33 |
| | Water-soluble ginger extract (K) | 0.5 | 0.5 | 0.5 | 0.5 |
| | Hiba arborvitae extract | 2.0 | 2.0 | 2.0 | 2.0 |
| | Methylparaben | 0.33 | 0.33 | 0.33 | 0.33 |
| | Succinic acid | 0.1 | 0.1 | 0.1 | 0.1 |
| | Sodium hydroxide solution (48%) | 0.07 | 0.07 | 0.07 | 0.07 |
| (F) | Water | 38.77 | 36.47 | 38.47 | 39.52 |
| | Total | 100.0 | 100.0 | 100.0 | 100.0 |
| | Component (A) | 8.0 | 8.0 | 8.0 | 8.0 |
| | Component (B) | 2.0 | 2.0 | 2.0 | 2.0 |
| | Component (C) | 1.7 | 4.0 | 2.0 | 0.95 |
| | Component (D) | 8.0 | 8.0 | 8.0 | 8.0 |
| | Component (E) | 8.0 | 8.0 | 8.0 | 8.0 |
| | Component (F) | 38.8 | 36.5 | 38.5 | 39.5 |
| | Viscosity (Pa·s) | 48 | 58 | 121 | 85 |

(continued)

| | | Example | | | |
|---|---|---|---|---|---|
| Component | Component name | 13 | 14 | 15 | 16 |
| Evaluation | Water evaporation suppression rate (%) | 49 | 45 | 42 | 47 |
| | Skin-covering feeling | 5.0 | 5.0 | 4.5 | 5.0 |
| | Glossiness of skin | 5.0 | 5.0 | 5.0 | 5.0 |
| | No stickiness and no uncomfortable feeling | 5.0 | 2.5 | 4.3 | 5.0 |
| | Storage stability (room temperature, 1 day) | A | A | A | A |
| | Storage stability (room temperature, 1 month) | A | A | A | B |

*1: Sphingolipid E (Kao Corporation)
*2: GE-IS (U) (HLB 2) (Kao Corporation)
*3: Silicone KF-6015 (PEG-3 dimethicone, HLB 4.5 (Shin-Etsu Chemical Co., Ltd.)
*4: Cosmol 42V (polyglyceryl-2 diisostearate) (Nisshin Oilio Group Ltd.)
*5: Silicone KF-96A-2CS (Shin-Etsu Chemical Co., Ltd.)
*6: Silicone KF-96A-6CS (Shin-Etsu Chemical Co., Ltd.)
*7: Silicone KSG-16 (Shin-Etsu Chemical Co., Ltd.)
*8: ELDEW CL-202 (Ajinomoto Co., Inc.)
*9: ELDEW PS-203 (Ajinomoto Co., Inc.)
*10: Plandool-H (Nippon Fine Chemical Co., Ltd.)
*11: Rheopearl KL2 (Chiba Flour Milling Co., Ltd.)
*15: Emulex HC-5 (PEG-5 hydrogenated castor oil, HLB 5 (Nippon Emulsion Co., Ltd.)
*21: Silicone KF-6017 (PEG-10 dimethicone, HLB5 (Shin-Etsu Chemical Co., Ltd.)
*22: Silicone KF-6028 (PEG-9 polydimethylsiloxyethyldimethicone, HLB 4) (Shin-Etsu Chemical Co., Ltd.)

Table 5

| | | Examples | | | Comparative Example | | |
|---|---|---|---|---|---|---|---|
| Componen t | Component name | 17 | 18 | 19 | 5 | 6 | 7 |
| (A) | N-(hexadecyloxyhydroxypropyl)-N-hydroxyethylhexadecanamide *1 | 8.0 | 3.0 | | 8.0 | | 3.0 |
| | Stearyl alcohol | | | 6.0 | | | |
| (C) | Polyoxyethylene methylpolysiloxane copolymer *3 | 1.5 | 1.5 | 1.5 | 1.5 | 1.5 | 1.5 |
| | Polyoxyethylene methylpolysiloxane copolymer *21 | | | 0.5 | | | |
| (B) | Polyglyceryl diisostearate *4 | 5.5 | | 2.0 | | 5.5 | |
| | Polyglyceryl triisostearate *12 | | 2.0 | | | | 2.0 |
| (E) | Squalane | 8.0 | 8.0 | 8.0 | 8.0 | 8.0 | 8.0 |
| | Methylpolysiloxane *5 | 8.0 | 8.0 | 8.0 | 8.0 | 8.0 | 8.0 |
| | Methylpolysiloxane *6 | 4.0 | 4.0 | 4.0 | 4.0 | 4.0 | 4.0 |
| | Methylpolysiloxane-crosslinked methylpolysiloxane mixture *7 | 5.0 | 5.0 | 5.0 | 5.0 | 5.0 | 5.0 |
| (D) | Dextrin Palmitate *11 | 0.5 | 0.5 | 0.5 | 0.5 | 0.5 | |
| | Magnesium sulfate heptahydrate | 0.7 | 0.7 | 0.7 | 0.7 | 0.7 | 0.7 |
| | Glycerol (86%) | 10.0 | 10.0 | 10.0 | 10.0 | 10.0 | 10.0 |
| | 1,3-Butylene glycol | 6.0 | 6.0 | 6.0 | 6.0 | 6.0 | 6.0 |

(continued)

| | | Examples | | | Comparative Example | | |
|---|---|---|---|---|---|---|---|
| Componen t | Component name | 17 | 18 | 19 | 5 | 6 | 7 |
| | Methylparaben | 0.33 | 0.33 | 0.33 | 0.33 | 0.33 | 0.33 |
| | Succinate | 0.10 | 0.10 | 0.10 | 0.10 | 0.10 | 0.10 |
| | Sodium hydroxide solution (48%) | 0.07 | 0.07 | 0.07 | 0.07 | 0.07 | 0.07 |
| (F) | Water | 42.3 | 50.8 | 47.3 | 47.8 | 50.3 | 51.3 |
| | Total | 100.0 | 100.0 | 100.0 | 100.0 | 100.0 | 100.0 |
| | Component (A) | 8.0 | 3.0 | 6.0 | 8.0 | 0.0 | 3.0 |
| | Component (B) | 5.5 | 2.0 | 2.0 | 0.0 | 5.5 | 2.0 |
| | Component (C) | 1.5 | 1.5 | 2.0 | 1.5 | 1.5 | 1.5 |
| | Component (D) | 0.5 | 0.5 | 0.5 | 0.5 | 0.5 | 0.0 |
| | Component (E) | 8.0 | 8.0 | 8.0 | 8.0 | 8.0 | 8.0 |
| | Component (F) | 42.3 | 50.8 | 47.3 | 47.8 | 50.3 | 51.3 |
| | Viscosity (Pa·s) | 65 | 74 | 70 | 65 | 65 | 55 |
| Evaluation | Water evaporation suppression rate (%) | 42 | 19 | 16 | 27 | 7 | 9 |
| | Skin-covering feeling | 4.5 | 3.0 | 2.8 | 2.5 | 1.8 | 1.8 |
| | Glossiness of skin | 4.3 | 3.3 | 3.3 | 1.8 | 3.8 | 2.8 |
| | No stickiness and no uncomfortable feeling | 3.5 | 3.8 | 2.3 | 3.8 | 3.8 | 4.0 |
| | Storage stability (room temperature, 1 day) | A | A | A | A | A | A |
| | Storage stability (room temperature, 1 month) | A | A | A | A | B | A |

*1: Sphingolipid E (manufactured by Kao Corporation)
*3: Silicone KF-6015 (PEG-3 dimethicone, HLB 4.5 (Shin-Etsu Chemical Co., Ltd.)
*4: Cosmol 42V (polyglyceryl-2 diisostearate) (Nisshin Oilio Group Ltd.)
*5: Silicone KF-96A-2CS (Shin-Etsu Chemical Co., Ltd.)
*6: Silicone KF-96A-6CS (Shin-Etsu Chemical Co., Ltd.)
*7: Silicone KSG-16 (Shin-Etsu Chemical Co., Ltd.)
*11: Rheopearl KL2 (Chiba Flour Milling Co., Ltd.)
*12: Cosmol 43V (polyglyceryl-2 triisostearate) (Nisshin Oilio Group Ltd.)
*21: Silicone KF-6017 (PEG-10 dimethicone, HLB 5) (Shin-Etsu Chemical Co., Ltd.)

Table 6

| | | Example | | |
|---|---|---|---|---|
| Component | Component name | 1 | 20 | 21 |
| | Allantoin | 0.5 | 0.5 | 0.5 |
| (A) | N-(hexadecyloxyhydroxypropyl)-N-hydroxyethylhexadecanamide *1 | 8.0 | 8.0 | |
| | Ceramide-2 | | | 8.0 |
| (C) | Isostearyl glyceryl ether *2 | 0.2 | 0.2 | 0.2 |
| | Polyoxyethylene/methylpolysiloxane copolymer *3 | 1.5 | 1.5 | 1.5 |
| (B) | Polyglyceryl diisostearate *4 | 2.0 | 2.0 | 2.0 |

(continued)

| | | Example | | |
|---|---|---|---|---|
| Component | Component name | 1 | 20 | 21 |
| (E) | Squalane | 8.0 | | |
| | Liquid isoparaffin *23 | | 8.0 | 8.0 |
| | Methylpolysiloxane *5 | 8.0 | 8.0 | 8.0 |
| | Methylpolysiloxane *6 | 4.0 | 4.0 | 4.0 |
| | Methylpolysiloxane-crosslinked methylpolysiloxane mixture *7 | 5.0 | 5.0 | 5.0 |
| (G) | Di(cholesteryl/octyldodecyl) N-lauroyl-L-glutamate *8 | 1.0 | 1.0 | 1.0 |
| | Di(phytosteryl/2-octyldodecyl) N-lauroyl-L-glutamate *9 | 2.0 | 2.0 | 2.0 |
| | Di(phytosteryl/isostearyl/cetyl/stearyl/behenyl) dilinolate *10 | 2.5 | 2.5 | 2.5 |
| (D) | Dextrin palmitate *11 | 0.5 | 0.5 | 0.5 |
| | Magnesium sulfate heptahydrate | 0.7 | 0.7 | 0.7 |
| | Glycerin (86%) | 10.0 | 10.0 | 10.0 |
| | 1,3-Butylene glycol | 4.0 | 4.0 | 4.0 |
| | Eucalyptus extract | 0.33 | 0.33 | 0.33 |
| | Water-soluble ginger extract (K) | 0.5 | 0.5 | 0.5 |
| | Hiba arborvitae extract | 2.0 | 2.0 | 2.0 |
| | Methylparaben | 0.33 | 0.33 | 0.33 |
| | Succinic acid | 0.1 | 0.1 | 0.1 |
| | Sodium hydroxide solution (48%) | 0.07 | 0.07 | 0.07 |
| (E) | Water | 38.77 | 38.77 | 38.77 |
| | Total | 100.0 | 100.0 | 100.0 |
| | Component (A) | 8.0 | 8.0 | 8.0 |
| | Component (B) | 2.0 | 2.0 | 2.0 |
| | Component (C) | 1.7 | 1.7 | 1.7 |
| | Component (D) | 0.5 | 0.5 | 0.5 |
| | Component (E) | 8.0 | 8.0 | 8.0 |
| | Component (F) | 38.8 | 38.8 | 38.8 |
| | Viscosity(Pa·s) | 48 | 49 | 93 |

(continued)

| | | Example | | |
|---|---|---|---|---|
| Component | Component name | 1 | 20 | 21 |
| Evaluation | Water evaporation suppression rate (%) | 49 | 46 | 43 |
| | Skin-covering feeling | 5.0 | 5.0 | 4.3 |
| | Glossiness of skin | 5.0 | 4.8 | 3.8 |
| | No stickiness and no uncomfortable feeling | 5.0 | 5.0 | 4.8 |
| | Storage stability (room temperature, 1 day) | A | A | A |
| | Storage stability (room temperature, 1 month) | A | A | A |
| *1: Sphingolipid E (Kao Corporation)<br>*2: GE-IS (U) (HLB 2) (Kao Corporation)<br>*3: Silicone KF-6015 (PEG-3 dimethicone, HLB 4.5 (Shin-Etsu Chemical Co., Ltd.)<br>*4: Cosmol 42V (Nisshin Oilio Group Ltd.)<br>*5: Silicone KF-96A-2CS (Shin-Etsu Chemical Co., Ltd.)<br>*6: Silicone KF-96A-6CS (Shin-Etsu Chemical Co., Ltd.)<br>*7: Silicone KSG-16 (Shin-Etsu Chemical Co., Ltd.)<br>*8: ELDEW CL-202 (Ajinomoto Co., Inc.)<br>*9: ELDEW PS-203 (Ajinomoto Co., Inc.)<br>*10: Plandool-H (Nippon Fine Chemical Co., Ltd.)<br>*11: Rheopearl KL2 (Chiba Flour Milling Co., Ltd.)<br>*23: PARLEAM EX (NOF Corporation) | | | | |

## Claims

**1.** A water-in-oil emulsion composition comprising the following components (A), (B), (C), (D), (E) and (F) :

(A) 1% by mass or more and 30% by mass or less of an oily component having a melting point of from 50 to 150°C;
(B) 0.5% by mass or more and 8% by mass or less of a polyglycerol fatty acid ester;
(C) a lipophilic surfactant other than component (B), having an HLB of from 2 to 6;
(D) 0.15% by mass or more and 8% by mass or less of a dextrin fatty acid ester having 8 to 22 carbon atoms in the fatty acid moiety;
(E) a hydrocarbon oil that is liquid at 25°C; and
(F) water.

**2.** The water-in-oil emulsion composition of claim 1, wherein the component (A) is a ceramide.

**3.** The water-in-oil emulsion composition according to claim 1 or 2, wherein the component (B) is one or more selected from the group consisting of a polyglycerol difatty acid ester and a polyglycerol trifatty acid ester.

**4.** The water-in-oil emulsion composition according to any one of claims 1 to 3, wherein the component (C) is a polyether modified silicone.

**5.** The water-in-oil emulsion composition according to any one of claims 1 to 4, wherein the water-in-oil emulsion composition has a viscosity at 25°C of 15 Pa·s or more and 5000 Pa·s or less and a cream form.

**6.** The water-in-oil emulsion composition according to any one of claims 1 to 5, wherein the content of the component (A) is 1.5% by mass or more and 20% by mass or less in the water-in-oil emulsion composition.

**7.** The water-in-oil emulsion composition according to any one of claims 1 to 6, wherein the content of the component (B) is 0.7% by mass or more and 7% by mass or less in the water-in-oil emulsion composition.

**8.** The water-in-oil emulsion composition according to any one of claims 1 to 7, wherein the content of the component

(D) is 0.2% by mass or more and 6% by mass or less in the water-in-oil emulsion composition.

**9.** The water-in-oil emulsion composition according to any one of claims 1 to 8, wherein the total content of the component (B) and the component (C) is 0.5% by mass or more and 12% by mass or less in the water-in-oil emulsion composition.

**10.** The water-in-oil emulsion composition according to any one of claims 1 to 9, wherein the component (A) is a compound represented by the formula (1)

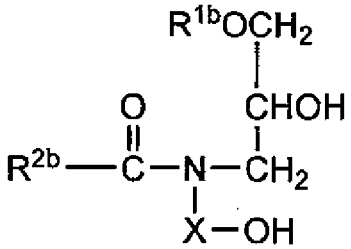

(1)

wherein $R^{1b}$ represents a hydrocarbon group having 10 to 26 carbon atoms, $R^{2b}$ represents a hydrocarbon group having 9 to 25 carbon atoms, and X represents $-(CH_2)_n-$ (where n represents an integer of from 2 to 6).

**11.** The water-in-oil emulsion composition according to any one of claims 1 to 10, wherein the component (D) is a dextrin fatty acid ester having 12 to 18 carbon atoms in the fatty acid moiety.

**12.** The water-in-oil emulsion composition according to any one of claims 1 to 11, wherein the component (E) is a squalane.

**13.** The water-in-oil emulsion composition according to any one of claims 1 to 12, wherein the water-in-oil composition has a viscosity at 25°C of 20 Pa·s or more and 1000 Pa·s or less and a cream form.

## INTERNATIONAL SEARCH REPORT

International application No.
PCT/JP2018/020797

A. CLASSIFICATION OF SUBJECT MATTER
Int.Cl. A61K8/68(2006.01)i, A61K8/06(2006.01)i, A61K8/31(2006.01)i, A61K8/37(2006.01)i, A61K8/73(2006.01)i, A61K8/894(2006.01)i, A61Q19/00(2006.01)i

According to International Patent Classification (IPC) or to both national classification and IPC

B. FIELDS SEARCHED
Minimum documentation searched (classification system followed by classification symbols)
Int.Cl. A61K8/68, A61K8/06, A61K8/31, A61K8/37, A61K8/73, A61K8/894, A61Q19/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2013-107865 A (ASANUMA CORPORATION) 06 June 2013, claims, paragraphs [0001]-[0011], [0019]-[0040], examples (Family: none) | 1, 3, 5-9, 11-13<br>1-13 |
| X<br>Y | JP 2013-14544 A (MIKIMOTO PHARMACEUT CO., LTD.) 24 January 2013, claims, paragraphs [0001]-[0017], examples (Family: none) | 1-2, 5, 7-9, 11-13<br>1-13 |
| X<br>Y | JP 2011-111401 A (KAO CORP.) 09 June 2011, claims, paragraphs [0001]-[0033], examples & US 2012/0301523 A1, claims, paragraphs [0001]-[0126], examples & WO 2011/065438 A1 & EP 2505182 A1 & CN 102665655 A & KR 10-2012-0116912 A | 1-13<br>1-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 August 2018 (24.08.2018) | 04 September 2018 (04.09.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/020797

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2011/118497 A1 (THE NISSHIN OILLIO GROUP, LTD.) 29 September 2011, claims, paragraphs [0001], [0016], [0070], examples & US 2013/0005808 A1, claims, paragraphs [0001], [0039], [0109], examples & EP 2550954 A1 & CN 102821743 A & KR 10-2013-0018240 A | 1-13 |
| Y | WO 99/25310 A1 (FUJISAWA PHARMACEUTICAL CO., LTD.) 27 May 1999, claims, pages 1-7, examples & US 6348479 B1, claims, pages 1-4, examples & EP 1029528 A1 & KR 10-2001-0031712 A & CN 1285732 A & JP 3509108 B2 | 1-13 |
| Y | JP 2011-21007 A (KAO CORP.) 03 February 2011, claims, paragraphs [0001]-[0042], examples & EP 2263639 A2, claims, paragraphs [0001]-[0057], examples & CN 101926736 A | 1-13 |
| Y | JP 2002-363029 A (KOSÉ CORPORATION) 18 December 2002, claims, paragraphs [0013]-[0020] (Family: none) | 1-13 |
| A | JP 2003-119107 A (KOKYU ALCOHOL KOGYO CO., LTD.) 23 April 2003, entire text (Family: none) | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9925310 A1
- JP 2016190816 A
- JP 2010513221 A
- JP S62228048 A
- JP S63216812 A
- JP S63227513 A
- JP S6429347 A
- JP S6431752 A
- JP H8319263 A